# EUROPEAN PATENT APPLICATION

(11) **EP 1 121 940 A1**
(43) Date of publication of application: **08.08.2001**
(21) Application number: 00101857.1
(22) Date of filing: 31.01.2000
(51) Int. Cl.: A61K 47/10, A61K 31/495, A61P 31/04, A61K 31/435

(54) **Pharmaceutical compositions comprising an antibiotic and bromhexine, and process for their preparation**

(71) Applicant: New Pharma Research Sweden AB, 22476 Lund (SE)
(72) Inventor: Shoa'a, Abdul Rahman, 22476 Lund (SE)
(74) Representative: Harrison, Michael Charles

(57) **Abstract**

Compositions for the prophylactic or therapeutic treatment of bacterial diseases in humans and animals, comprising as essential active ingredients a quinolone- or naphtyridone-type antibiotic or an active chemical derivative thereof and bromhexine-HCl, glacial acetic acid, water and a stabilizing agent. The compositions are stable upon storage over a broad range of temperature. The invention concerns also the combined use of a quinolone- or naphtyridone-type antibiotic or an active chemical derivative thereof and bromhexine-HCl for the treatment of bacterial infections in humans and animals, and a process for the preparation of the aforementioned compositions.

## Description

### Field of the invention:

The present invention concerns aqueous compositions comprising an antibiotic, the compositions being intended to be used in the pharmaceutical field, and preferably for the prophylaxis or treatment of bacterial diseases affecting humans or farm and domestic animals. The present invention concerns also a process for the preparation of the compositions.

### Background of the invention:

It is known that quinolone- or naphtyridone-type antibiotics are useful as agents for the treatment of bacterial diseases affecting humans or animals. Antibiotics belonging to this group are for example enrofloxacin, ciprofloxacin, ofloxacin, flumequine, norfloxacin, nalidixic acid, cinoxacin, clinafloxacin, difloxacin, enoxacin, fleroxacin, miloxacin, nadifloxacin, pefloxacin, pipemidic acid, rosoxacin, rufloxacin, sparfloxacin, temafloxacin, tosufloxacin, trovafloxacin, orbifloxacin, marbofloxacin, benofloxacin, binfloxacin, danofloxacin, sarafloxacin, premafloxacin or ibafloxacin.

Enrofloxacin is sold, for example, by the company Bayer AG, Germany, under the trade name Baytril®. Enrofloxacin is the generic name of a fluoroquinolone carboxylic compound. More specifically, enrofloxacin is 1-cyclopropyl-7-(4-ethyl-1-piperazinyl)-6-fluoro-1,4-dihydro-4-oxo-3-quinoline carboxylic acid.

Suitable salts of quinolones are those of inorganic or organic salts selected from hydrochloric acid, hydrobromic acid, hydroiodic acid, sulfuric acid, phosphoric acid, acetic acid, succinic acid, phosphonic acid, malic acid, sodium hydroxyde, potassium hydroxyde, aluminium hydroxyde, piperidine, morpholine, ethylamine and triethylamine.

Several studies have been conducted to improve the efficacy of the antibiotic treatment. The following examples show in which direction the studies were conducted.

Bonse et al. (US Patent 5,998,418) describes injection and infusion aqueous solutions based on the salt of enrofloxacin with specific polycarboxylic acids or amino-acids, for combatting bacteria in an animal. The pH of the aqueous solution is 3 to 5.5. According to the author, the advantage of these solutions is that the active substance is present in high levels in the blood only a short time after administration and that it is eliminated rapidly.

Also, Copeland et al. (US Patent 5,756,506) describes a process for the treatment of bacterial infections like the bovine respiratory disease or the swine pleuropneumonia, by administering by injection a composition containing a single high dose of between 7.5 and 15 mg/kg of enrofloxacin to the animal. According to the author, the advantage is that the single high dose treatment can replace repeated treatments.

In another reference, Vetter et al. (US Patent 5,808,076) describes orally administrable formulations containing a quinolone- or naphtyridone-carboxylic acid embonate and an excipient, like for example corn starch, colloidal silica, vaseline or paraffin (used as a carrier). According to the author, the presence of embonic acid in the formulation has the advantage of masking the bitter taste of the quinolone- or naphtyridone-carboxylic acid and therefore improves the acceptance of the orally administrable formulation by the subject animal.

From the above examples, it can be concluded that to improve the efficacy of the antibiotic treatment, either the authors have tried to change the posology (changing the dose of antibiotic injected), or they have tried to improve the absorption or the acceptance of the substance by the subject animal. In the latter case, further compounds were added in the composition.

Other studies to improve the effect of the antibiotic treatment have been made, such as for example by combination therapy (a treatment whereby several agents are administered separately but simultaneously to the patient).

Schmidt et al. (Tierärtzl. Prax. 1998, 26(G), pages 127-132) describes such a combination therapy with enrofloxacin and with a bronchosecretolytic agent, bromhexine-HCl.

Bromhexine-HCl is the generic name of N-(2-amino-3,5-dibromobenzyl)-N-methyl-cyclohexylamine hydrochloride and is known as an anti-histamine, expectorant, mucolytic drug. It is sold, for example, by the company Boehringer Ingelheim Vetmedia GmbH, Germany, under the trade name Bisolvon®, in a dry form or as a 0.2% solution. Bromhexine-HCl is known to precipitate in solutions of pH greater than six.

The aim of the clinical studies of Schmidt et al. was to determine the clinical efficacy of a combined treatment with enrofloxacin and bromhexine-HCl in respiratory diseased bovines, whereby during the treatment bromhexine-HCl was in a first stage injected parenterally and in a second stage given orally. According to the authors, an acceleration of the recovery of the animals treated by the combination therapy was observed when compared to the treatment with the antibiotic alone.

In other respects, it is known from the literature (see Kern et al., US Patent 5,808,076) to combine bromhexine-HCl and erythromycin, erythromycin lactobionate, tylosin, oxytetracycline-HCl, ampicillin or benzathine ampicillin, to improve the absorption of the antibacterial substance. Other antibiotics are cited in this reference which could, according to the author, also be combined either with bromhexine-HCl or with another benzylamine derivative, ambroxol. These antibiotics belong to the tetracycline, beta-lactam, erythromycin, spiramycin, tylosin, oleandomycin, chloramphenicol, thiamphenicol or sulfonamide groups.

However, the prior art does not disclose a composition which combines both an antibiotic of the quinolone- or naphtyridone-type, like enrofloxacin, and bromhexine-HCl.

Studies made by the present inventors have shown that bromhexine-HCl cannot be solubilized in the usual antibiotic compositions containing a quinolone- or naphtyridone-type antibiotic like enrofloxacin.

Surprisingly, the inventors of the present invention have found that an aqueous stable composition comprising as essential active ingredients enrofloxacin and bromhexine-HCl is achieved when glacial acetic acid and a stabilizing agent is added to the composition, the stabilizing agent being preferably selected from benzyl alcohol, N-methylpyrrolidone, 2-pyrrolidone, dimethylacetamate (DMA), dimethylformamide (DMF), dimethylsulfoxide (DMSO) or Tween-80, or a combination thereof.

A stable composition in the meaning of the present invention is a composition wherein the compounds are present in a soluble form, i.e. there is no precipitate, the composition remaining stable during storage (at least two months) at temperatures comprised between about +2°C and +60°C. Hence, the composition can be used in countries where the possibility of keeping it in a stable temperature environment is not guaranteed.

The inventors of the present invention have also found that the same effect is obtained when enrofloxacin is replaced by any other quinolone- or naphtyridone-type antibiotic or derivative thereof.

The inventors of the present invention have also found a process for the preparation of said compositions.

The inventors of the present invention have also found that said compositions are effective for the prophylaxis or treatment of diseases in humans and animals, in particular the treatment of bacterial infections due to microorganisms pathogenic to humans and animals.

Thus, the main object of the present invention is to provide new compositions which are effective as antibacterial agents.

Another object of the present invention is to provide these compositions as compositions which are stable during storage over a wide range of temperature.

Another object of the present invention is to provide these compositions as compositions which can be administered orally or by injection.

Another object of the present invention is to provide a process for the preparation of these compositions.

One of the advantages of the present invention is therefore that the composition can be stored in a stable manner in the form in which it will be administered.

Another advantage of the present invention is that the composition has better absorption characteristics than a composition which contains only the antibiotic.

Further problems which can be solved by this invention with respect to known prior art compositions will become apparent to the reader of the following description.

### Summary of the invention:

The present invention provides a solution to the aforementioned problems.

The aforementioned object is achieved by a composition having the features defined in claim 1 and comprising as essential active ingredients a quinolone- or naphtyridone-type antibiotic or derivative thereof and bromhexine-HCl, glacial acetic acid, water and a stabilizing agent, the stabilizing agent being selected from benzyl alcohol, N-methylpyrrolidone, 2-pyrrolidone, dimethylacetamate (DMA), dimethylformamide (DMF), dimethylsulfoxide (DMSO) or Tween-80, or a combination thereof.

The aforementioned object is also achieved by the use of the compositions for the prophylaxis or treatment of diseases, and most preferably bacterial infections affecting humans or animals.

Preferred embodiments of the invention, including the use of chemical derivatives of the essential compounds of the composition or preferred concentrations, the addition of optional ingredients in the composition like for example formalin, excipients, preservatives, vitamins, further stabilizing agents, carriers, antioxidants, photostabilizers, colorants, other absorption-promoting substances, or the use of the composition for the treatment of specific bacterial diseases in humans or animal are defined in the dependent claims.

The aforementioned object is also achieved by the process for the preparation of the composition comprising: (i) in a first stage the preparation of solution A comprising the dispersion of bromhexine-HCl in part of the glacial acetic acid, the addition of the stabilizing agent(s) followed by the addition of part of the water, and the mixing and heating until bromhexine-HCl is completely dissolved; (ii) in a second stage the preparation of solution B comprising the dissolution of the quinolone- or naphtyridone-type antibiotic or derivative thereof in the rest of the glacial acetic acid and water by simple mixing at room temperature; (iii) in a third stage the mixing of A and B and homogenisation, followed by the filtration of the composition through a 60 micron filter, followed by filtration through a 6 micron filter. In the case of a formulation intended to be injected, further filtration through a 0.1 micron filter is carried out.

The aforementioned object is also achieved by an alternative process for the preparation of the composition wherein bromhexine-HCl is first dissolved in the whole amount of glacial acetic acid, followed by the addition of the stabilizing agent(s), the addition of water, the mixing and heating until until bromhexine-HCl is completely dissolved, and then the addition of the quinolone- or naphtyridone-type antibiotic or derivative thereof and mixing until dissolution, followed by the same filtration procedure as above, with the proviso that if Tween-80 is added as the stabilizing agent it is added only after the antibiotic.

Unless otherwise specified, the percentages of ingredients used in the compositions are defined as weight per volume. The preferred concentrations or ranges of concentrations mentioned hereafter are concentrations which are more suitable to achieve the object of the present invention.

### Description of preferred embodiments:

The composition according to the present invention comprises as essential active ingredients a quinolone- or naphtyridone-type antibiotic or an active chemical derivative thereof and bromhexine-HCl, glacial acetic acid, water and at least one stabilizing agent.

The quinolone- or naphtyridone-type antibiotic is preferably selected from enrofloxacin, ciprofloxacin, ofloxacin, flumequine, norfloxacin, nalidixic acid, cinoxacin, clinafloxacin, difloxacin, enoxacin, fleroxacin, miloxacin, nadifloxacin, pefloxacin, pipemidic acid, rosoxacin, rufloxacin, sparfloxacin, temafloxacin, tosufloxacin, trovafloxacin, orbifloxacin, marbofloxacin, benofloxacin, binfloxacin, danofloxacin, sarafloxacin, premafloxacin or ibafloxacin. Enrofloxacin, ciprofloxacin, norfloxacin, danofloxacin or sarafloxacin will preferably be used.

The amount of quinolone- or naphtyridone-type antibiotic or its active chemical derivative in the composition is preferably between 5 and 40% weight/volume. Most preferably, the composition contains 20% weight/volume of quinolone- or naphtyridone-type antibiotic or its active chemical derivative.

The amount of bromhexine-HCl in the composition is preferably between 1 and 4% weight/volume. Most preferably, the composition contains 1% weight/volume of bromhexine-HCl.

The amount of glacial acetic acid in the composition is preferably between 5 and 16% weight/volume. Most preferably, the composition contains 8% weight/volume of glacial acetic acid.

The amount of water in the composition is preferably superior or equal to 35% weight/volume.

The stabilizing agent can be selected from benzyl alcohol, N-methylpyrrolidone, 2-pyrrolidone, dimethylacetamate (DMA), dimethylformamide (DMF), dimethylsulfoxide (DMSO) or Tween-80, or a combination thereof.

Preferably, the stabilizing agent is a combination of Tween-80 and 2-pyrrolidone, Tween-80 and N-methylpyrrolidone, Tween-80 and dimethylacetamate (DMA), Tween-80 and dimethylformamide (DMF), Tween-80 and dimethylsulfoxide (DMSO), or Tween-80, 2-pyrrolidone and benzyl alcohol.

The total amount of stabilizing agent in the composition is preferably between 9 and 30% weight/volume.

The amount of Tween-80 in the composition is preferably between 5 and 20% weight/volume. Most preferably, 10% weight/volume of Tween-80 will be used.

The amount of N-methylpyrrolidone, 2-pyrrolidone, dimethylacetamate (DMA), dimethylformamide (DMF) or dimethylsulfoxide (DMSO) in the composition is preferably between 2 and 20% weight/volume. Most preferably, 4% weight/volume of one of these agents will be used.

The amount of benzyl alcohol in the composition is preferably between 1 and 5% weight/volume.

Variations of the amount of stabilizing agent depend on the combination used and on the amount of antibiotic and bromhexine-HCl in the composition.

Preferably, when the composition comprises 20% weight/volume of enrofloxacin and 1% weight/volume of bromhexine-HCl, a combination of 4% weight/volume of 2-pyrrolidone and 10% weight/volume of Tween-80 will be used as stabilizing agent.

Optionally, formalin may be added in the compositions intended to be used for oral administration, since the inventors of the present invention have discovered that an addition of formalin allows a better solubilization of bromhexine-HCl and improves the stability of the composition during storage. The amount of formalin added in the composition is preferably 3% weight/volume.

Formalin is the name of a 37% (w/w) aqueous solution of formaldehyde. This solution, which can be purchased from various sources, contains usually between less than 1% to 15% of methanol and less than 0.05% (w/w) of formic acid.

The compositions may also comprise optionally other agents like excipients, preservatives, vitamins, further stabilizing agents, carriers, antioxidants, photostabilizers, colorants, other absorption-promoting substances, thickeners or any other agent or additive commonly used in veterinary or medical compositions which is for example useful for the stability of the composition or permits to improve the formulation for a specific way of administration without altering the antibacterial activity.

When necessary, the composition is generally complemented to the required final volume by the addition of deionized water.

The process for the preparation of the composition comprises: (i) in a first stage the preparation of a solution A comprising the dispersion of bromhexine-HCl in part of the glacial acetic acid, the addition of the stabilizing agent(s) followed by the addition of part of the water, and the mixing and heating until bromhexine-HCl is completely dissolved; (ii) in a second stage the preparation of a solution B comprising the dissolution of the quinolone- or naphtyridone-type antibiotic or derivative thereof in the rest of the glacial acetic acid and water by simple mixing at room temperature; (iii) in a third stage the mixing of A and B and the homogenisation of the obtained composition, followed by the filtration of the composition through a 60 micron filter, followed by filtration through a 6 micron filter. In the case of a formulation intended to be injected, further filtration through a 0.1 micron filter is carried out to ensure that no particles of a size higher than 0.1 micron, like e.g. microorganisms, are present in the composition.

The parts of the glacial acetic acid and water used in solution A represent preferably about 40% of the total amount of each of these components present in the final composition.

An alternative process for the preparation of the composition is possible, wherein bromhexine-HCl is first dissolved in the whole amount of glacial acetic acid, followed by the addition of the stabilizing agent(s), the addition of water, the mixing and heating until bromhexine-HCl is completely dissolved, and then the addition of the quinolone- or naphtyridone-type antibiotic or derivative thereof and mixing until dissolution, followed by the same filtration procedure as above, with the proviso that if Tween-80 is added as stabilizing agent it is added only after the antibiotic in order to avoid an increase of the viscosity which would lead to a delay in the solubilization of the antibiotic.

In a preferred embodiment applicable to both alternate processes, the temperature up to which the solution is heated until bromhexine-HCl is completely dissolved is between about +50°C and +60°C. Most preferably a heating up to +60°C is carried out.

The optional agents like formalin will preferably be added at the end of the process, but before filtration.

Generally, the compositions are intended to be administered orally or by injection and will be prepared in a suitable form. However, it should be noted that the form in which the compositions are administered depends upon the particular bacterial infection to be treated and may be adapted in order, for example, to deliver the agent closer to the site of infection. Thus, in the case of infections of a topical nature, a formulation for topical application may be prepared and topical application may be used.

The compositions are effective for the prophylaxis or treatment of diseases in humans and animals, in particular the treatment of bacterial infections due to microorganisms pathogenic to humans and animals.

The microorganisms include, for example, microorganisms of the following types: streptococci, staphylococci, pseudomonas, enterobacteria as e.g. Escherichia coli or salmonella, listeria, clostridium, corynebacteria, mycobacteria, actinomycetes, rickettsia, chlamydia or mycoplasma.

The posology and way of administration depend on the subject to be treated and the stage and severity of the infection.

For example, for systemic treatment of cattle and calves suffering from pneumoniae, the composition is administered by intramuscular injection and the antibiotic is given at a daily dose of about 2.5 mg/kg body weight, one time each day and for generally between two and five successive days.

For systemic treatment of poultry infected by Escherichia coli, the composition is given orally, at a dose of 1 ml of composition per 4 litre of drinking water, and continuously for three consecutive days. This corresponds approximately to an antibiotic dose of 10 mg/kg body weight in the case of chicken.

The invention will now be described in more detail with reference to the following examples.

### Examples:

The amounts of ingredients are given in % of weight/volume. The volume is adjusted by addition of deionized water.

### Example 1:

A composition comprising:

| | |
|---|---|
| Enrofloxacin | 20% |
| Bromhexine-HCl | 1% |
| Acetic acid | 8% |
| Tween-80 | 10% |
| 2-pyrrolidone | 4% |
| Water | q.s.p. 100% |

### Example 2:

A process for the preparation of 100 ml of the composition of example 1 wherein:
(i) in a first stage a solution A is prepared, comprising the dispersion of 1 g of bromhexine-HCl in 3 g of glacial acetic acid, the addition of 4 g of 2-pyrrolidone and 10 g of Tween-80, followed by the addition of 22 g of water, and the mixing and heating up to + 60°C until bromhexine-HCl is completely dissolved;
(ii) in a second stage a solution B is prepared, comprising the dissolution of 20 g enrofloxacin in 5 g of glacial acetic acid and 35 g of water by simple mixing at room temperature;
(iii) in a third stage A and B are mixed together and homogenised, and the composition is filtrated through a 60 micron filter, followed by filtration through a 6 micron filter.

In the case of a formulation intended to be injected, further filtration through a 0.1 micron filter is carried out.

### Example 3:

A process for the preparation of 100 ml of the composition of example 1 wherein:
1 g of bromhexine-HCl is dissolved in 8 g of glacial acetic acid, followed by the addition of 4 g of 2-pyrrolidone, the addition of 57 g of water, the mixing and heating up to +60°C until bromhexine-HCl is completely dissolved, and then the addition of 20 g of enrofloxacin and mixing until dissolution, followed by the addition of 10 g of Tween-80, and followed by the same filtration procedure as in Example 2.

### Example 4:

A composition comprising:

| | |
|---|---|
| Enrofloxacin | 20% |
| Bromhexine-HCl | 1% |
| Acetic acid | 8% |
| Tween-80 | 5% |
| 2-pyrrolidone | 4% |
| Water | q.s.p. 100% |

### Example 5:

A composition comprising:

| | |
|---|---|
| Enrofloxacin | 20% |
| Bromhexine-HCl | 1% |
| Acetic acid | 6% |
| Tween-80 | 5% |
| 2-pyrrolidone | 4% |
| Water | q.s.p. 100% |

### Example 6:

A composition comprising:

| | |
|---|---|
| Enrofloxacin | 5% |
| Bromhexine-HCl | 1% |
| Acetic acid | 5% |
| Tween-80 | 7.5% |
| 2-pyrrolidone | 4% |
| Water | q.s.p. 100% |

### Example 7:

A composition comprising:

| | |
|---|---|
| Enrofloxacin | 10% |
| Bromhexine-HCl | 2% |
| Acetic acid | 8% |
| Tween-80 | 15% |
| 2-pyrrolidone | 4% |
| Water | q.s.p. 100% |

### Example 8:

A composition comprising:

| | |
|---|---|
| Enrofloxacin | 20% |
| Bromhexine-HCl | 1% |
| Acetic acid | 8% |
| Tween-80 | 8% |
| DMA | 4% |
| Water | q.s.p. 100% |

### Example 9:

A composition comprising:

| | |
|---|---|
| Enrofloxacin | 20% |
| Bromhexine-HCl | 1% |
| Acetic acid | 8% |
| Tween-80 | 10% |
| DMF | 4% |
| Water | q.s.p. 100% |

### Example 10:

A composition comprising:

| | |
|---|---|
| Enrofloxacin | 20% |
| Bromhexine-HCl | 1% |
| Acetic acid | 8% |
| Tween-80 | 10% |
| DMSO | 4% |
| Water | q.s.p. 100% |

### Example 11:

A composition comprising:

| | |
|---|---|
| Enrofloxacin | 20% |
| Bromhexine-HCl | 1% |
| Acetic acid | 8% |
| Tween-80 | 10% |
| 2-pyrrolidone | 4% |
| Benzyl alcohol | 5% |
| Water | q.s.p. 100% |

### Example 12:

A composition comprising:

| | |
|---|---|
| Enrofloxacin | 20% |
| Bromhexine-HCl | 1% |
| Acetic acid | 8% |
| Tween-80 | 10% |
| 2-pyrrolidone | 2% |
| Benzyl alcohol | 1% |
| Water | q.s.p. 100% |

### Example 13:

A composition comprising:

| | |
|---|---|
| Enrofloxacin | 30% |
| Bromhexine-HCl | 1.5% |
| Acetic acid | 16% |
| Tween-80 | 15% |
| 2-pyrrolidone | 8% |
| Benzyl alcohol | 5% |
| Water | q.s.p. 100% |

### Example 14:

A composition comprising:

| | |
|---|---|
| Enrofloxacin | 30% |
| Bromhexine-HCl | 1.5% |
| Acetic acid | 15% |
| Tween-80 | 20% |
| 2-pyrrolidone | 4% |
| Water | q.s.p. 100% |

### Example 15:

A composition comprising:

| | |
|---|---|
| Enrofloxacin | 40% |
| Bromhexine-HCl | 2% |
| Acetic acid | 10% |
| Tween-80 | 10% |
| 2-pyrrolidone | 5% |
| Water | q.s.p. 100% |

### Example 16:

A composition comprising:

| | |
|---|---|
| Enrofloxacin | 30% |
| Bromhexine-HCl | 1.5% |
| Acetic acid | 10% |
| Tween-80 | 10% |
| 2-pyrrolidone | 5% |
| Water | q.s.p. 100% |

### Example 17:

A composition comprising:

| | |
|---|---|
| Enrofloxacin | 20% |
| Bromhexine-HCl | 4% |
| Acetic acid | 10% |
| Tween-80 | 10% |
| 2-pyrrolidone | 20% |
| Water | q.s.p. 100% |

### Example 18:

A composition comprising:

| | |
|---|---|
| Enrofloxacin | 20% |
| Bromhexine-HCl | 1% |
| Acetic acid | 8% |
| Tween-80 | 10% |
| 2-pyrrolidone | 4% |
| Formalin (37%) | 3% |
| Water | q.s.p. 100% |

All the above exemplified compositions are stable upon storage at least for two months and over a wide range of temperature (between about +2°C and +60°C).

When used for the prophylaxis or treatment of bacterial diseases in humans and animals, the compositions in accordance with the present invention are more effective than compositions which do not contain the combination of the antibiotic and bromhexine-HCl.

The following examples illustrates the efficacy of the compositions of the present invention and their industrial applicability.

### Evaluation of the efficacy of a composition in accordance with the present invention in the treatment of Escherichia coli infected chickens.

The aim of this study was to evaluate the effect of a treatment with a composition in accordance with the present invention on the mortality and clinical signs of chickens naturally infected by Escherichia coli, and to compare the efficacy of this treatment with the efficacy of a treatment with a composition containing only the antibiotic.

Thus, the following experiment was carried out.

10 000 broiler chickens aged 1 day were used for the experiment. The birds were infected by natural infection by Escherichia coli.

The birds were then divided into 2 equal groups of 5 000 birds each. The first group was treated with a composition containing only enrofloxacin. The second group was treated with the composition of Example 1 of the present invention, containing both enrofloxacin and bromhexine-HCl. Thus, 1 ml of composition contained either 200 mg of enrofloxacin (composition for the treatment of the first group) or 200 mg of enrofloxacin and 10 mg of bromhexine-HCl (composition for the treatment of the second group).

For both groups, the treatment was made orally, for three consecutive days, and using the drinking water, at a dose of 1 ml of active composition per 4 litres of drinking water.

To assess the efficacy of the treatment, the mortality and body weight of the animals were recorded for each group over a period of ten days following the treatment. The results are summarized in the following Table I.

**Table I**

| **Treatment** | **Mortality** | | **Weight** | |
|---|---|---|---|---|
| | dead birds | % | g/bird | % |
| Enrofloxacine | 113/5000 | 2.23 | 1810 | -- |
| Enrofloxacine + bromhexine-HCl | 32/5000 | 0.6 | 1950 | 7.7 |

As is clear from Table I, the combined treatment with enrofloxacine and bromhexine-HCl is more effective in controlling the mortality associated with the bacterial infection than the treatment using only enrofloxacine. Furthermore, the group of birds treated with the combination of enrofloxacine and bromhexine-HCl showed a significant increase in body weight.

Therefore, the composition in accordance with the present invention has clearly a therapeutic effect on chickens infected by Escherichia coli.

### Evaluation of the efficacy of a composition in accordance with the present invention in the treatment of calves suffering from a pneumonia infection

The aim of this study was to evaluate the effect of a treatment with a composition in accordance with the present invention on the mortality and clinical signs of calves suffering from a pneumonia infection, and to compare the efficacy of this treatment with the efficacy of a treatment with a composition containing only the antibiotic.

Thus, the following experiment was carried out.

20 calves aged 1 to 4 months were used for the experiment. The animals were suffering from a pneumonia infection, characterized by severe respiratory disorders including coughing, rale and increased body temperature (measured between +39.5°C and +41°C).

The calves were divided into 2 equal groups of 10 animals each. The first group was treated with a composition containing only enrofloxacin. The second group was treated with the composition of Example 1 of the present invention, i.e. containing both enrofloxacin and bromhexine-HCl. Thus, 1 ml of composition contained either 200 mg of enrofloxacin (composition for the treatment of the first group) or 200 mg of enrofloxacin and 10 mg of bromhexine-HCl (composition for the treatment of the second group).

For both groups, the treatment was made by intramuscular injection, once a day and for four consecutive days, at a dose of 1 ml of active composition per 80 kg of animal body weight.

To assess the efficacy of the treatment, the mortality and body temperature of the animals were recorded for each group over a period of ten days following the treatment. The results after 5 days are summarized in the following Table II.

**Table II**

| **Treatment** | **Mortality** | | **Body temperature** |
|---|---|---|---|
| | dead animals | % | |
| Enrofloxacine | 1/10 | 10 | over 41.5°C |
| Enrofloxacine + bromhexine-HCl | 0/10 | 0 | returned to normal |

As is clear from Table II, the combined treatment with enrofloxacine and bromhexine-HCl is more effective in curing the disease associated with the infection than the treatment using only enrofloxacine, since after 5 days the body temperature of the animals treated with enrofloxacine and bromhexine-HCl returned to a normal value, whereas almost no change was observed for the animals which were treated with only the antibiotic. Furthermore, no animal died in the group treated with the combination of enrofloxacine and bromhexine-HCl.

Therefore, the composition in accordance with the present invention has clearly a therapeutic effect on calves suffering from an infection like pneumonia.

### Evaluation of the absorption-promoting effect of bromhexine-HCl on enrofloxacin in the compositions in accordance with the present invention

Experiments were made to measure the pharmaco-kinetic parameters of the antibacterial activity in the blood of animals treated with either a composition in accordance with the present invention or a composition containing only the antibiotic.

The aim of this study was to evaluate the absorption-promoting effect of bromhexine-HCl on enrofloxacin. The experiments were performed on chickens, sheep, goats and cattle, and the treatment was made either orally in the case of chickens or by intramuscular injection in the case of the other animals.

In all cases, the results showed a significant increase of the blood level of enrofloxacin when the antibiotic was given in combination with bromhexine-HCl. The absorption half-life of enrofloxacin was also shorter when the antibiotic was given in combination with bromhexine-HCl. These results demonstrate clearly an absorption-promoting effect of bromhexine-HCl on enrofloxacin.

The compositions according to the present invention can therefore be used as prophylaxis or treatment for animals or humans infected by bacterial diseases.

Although the present invention has been described with reference to several examples and embodiments of specific compositions and concentrations of ingredients, this is not to be considered as a limitation of the invention but merely illustrative thereof.

Specifically, other compounds like chemical derivatives of the active compounds cited herein could be used, as soon as the modification does not lead to a substantial loss of the activity of the compound. For example, derivatives of the quinolone- or naphtyridone-type antibiotics include their esters such as the C1-C4 alkyl esters.

## Claims

1. A composition comprising a quinolone- or naphtyridone-type antibiotic or an active chemical derivative thereof, bromhexine-HCl, a stabilizing agent, glacial acetic acid and water, the stabilizing agent being selected from benzyl alcohol, N-methylpyrrolidone, 2-pyrrolidone, dimethylacetamate (DMA), dimethylformamide (DMF), dimethylsulfoxide (DMSO) or Tween-80, or a combination thereof.

2. A composition according to claim 1 wherein the antibiotic is preferably selected from enrofloxacin, ciprofloxacin, norfloxacin, danofloxacin or sarafloxacin.

3. A composition according to claim 1 or 2 wherein the amount of antibiotic is between 5% and 40% weight/volume.

4. A composition according to any one of claims 1 to 3 wherein the amount of antibiotic is 20% weight/volume.

5. A composition according to any one of claims 1 to 4 wherein the amount of bromhexine-HCl is between 1% and 4% weight/volume.

6. A composition according to any one of claims 1 to 5 wherein the amount of bromhexine-HCl is 1% weight/volume.

7. A composition according to any one of claims 1 to 6 wherein the amount of acetic acid is between 5% and 16% weight/volume.

8. A composition according to any one of claims 1 to 7 wherein the amount of acetic acid is 8% weight/volume.

9. A composition according to any one of claims 1 to 8 wherein the amount of water is superior or equal to 35% weight/volume.

10. A composition according to any one of claims 1 to 9 wherein the stabilizing agent is a combination selected from the group consisting of Tween-80 and 2-pyrrolidone, Tween-80 and N-methylpyrrolidone, Tween-80 and dimethylacetamate (DMA), Tween-80 and dimethylformamide (DMF), Tween-80 and dimethylsulfoxide (DMSO), or Tween-80, 2-pyrrolidone and benzyl alcohol.

11. A composition according to any one of claims 1 to 10 wherein the amount of stabilizing agent is between 9% and 30% weight/volume.

12. A composition according to any one of claims 1 to 11 which comprises 20% weight/volume of enrofloxacin, 1% weight/volume of bromhexine-HCl, 4% weight/volume of 2-pyrrolidone and 10% weight/volume of Tween-80.

13. A composition according to any one of claims 1 to 12 which further comprises formalin.

14. A composition according to claim 13 wherein the amount of formalin is 3% weight/volume.

15. A composition according to any one of claims 1 to 14 which further comprises other agents like excipients, preservatives, vitamins, further stabilizing agents, carriers, antioxidants, photostabilizers, colorants, other absorption-promoting substances, or thickeners.

16. A composition according to any one of claims 1 to 15 for its use as a medicament.

17. Use of a composition according to any one of claims 1 to 16 for the preparation of a medicament for the prophylactic or therapeutic treatment of bacterial diseases.

18. Process for the preparation of a composition according to any one of claims 1 to 12 comprising: (i) in a first stage the preparation of a solution A comprising the dispersion of bromhexine-HCl in part of the glacial acetic acid, the addition of the stabilizing agent(s) followed by the addition of part of the water, and the mixing and heating until bromhexine-HCl is completely dissolved; (ii) in a second stage the preparation of a solution B comprising the dissolution of the quinolone- or naphtyridone-type antibiotic or derivative thereof in the rest of the glacial acetic acid and water by simple mixing at room temperature; (iii) in a third stage the mixing of A and B and the homogenisation of the obtained composition.

19. Process according to claim 18 wherein the part of the glacial acetic acid and water used in solution A represents about 40% of the total amount of each of these components present in the final composition.

20. Process for the preparation of a composition according to any one of claims 1 to 12 comprising the steps of first dissolving bromhexine-HCl in the whole amount of glacial acetic acid, followed by the addition of the stabilizing agent(s), the addition of water, the mixing and heating until bromhexine-HCl is completely dissolved, and then the addition of the quinolone- or naphtyridone-type antibiotic or derivative thereof and mixing until dissolution, with the proviso that if Tween-80 is added as stabilizing agent it is added only after the antibiotic.

21. Process according to any one of claims 18 to 20 wherein the temperature up to which the solution is heated until bromhexine-HCl is completely dissolved is between about +50°C and +60°C.

22. Process according to any one of claims 18 to 21 wherein the temperature up to which the solution is heated until bromhexine-HCl is completely dissolved is +60°C.

23. Process according to any one of claims 18 to 22 further comprising a step of filtration of the composition.
